# EUROPEAN PATENT SPECIFICATION

(11) **EP 4 105 882 B1**
(45) Date of publication and mention of the grant of the patent: **24.07.2024**
(21) Application number: 21788221.6
(22) Date of filing: 18.03.2021
(51) Int. Cl.: G06T 7/00, G16H 30/40, G16H 50/20, G06V 10/82

(54) **INFORMATION PROCESSING DEVICE, INFORMATION PROCESSING METHOD, AND COMPUTER PROGRAM**
INFORMATIONSVERARBEITUNGSVORRICHTUNG, INFORMATIONSVERARBEITUNGSVERFAHREN UND COMPUTERPROGRAMM
DISPOSITIF DE TRAITEMENT D'INFORMATIONS, PROCÉDÉ DE TRAITEMENT D'INFORMATIONS ET PROGRAMME INFORMATIQUE

(30) Priority: 14.04.2020 JP 2020072381
(43) Date of publication of application: 21.12.2022
(73) Proprietor: EIZO Corporation, Ishikawa 924-8566 (JP)
(72) Inventor: HIGASHI Masafumi, Hakusan-shi, Ishikawa 924-8566 (JP); KATO Yu, Hakusan-shi, Ishikawa 924-8566 (JP)
(74) Representative: McKinnon, Alistair James
(86) International application number: PCT/JP2021/010960
(87) International publication number: WO 2021/210334

(56) References cited:
- WO-A1-2020/039968
- WO-A1-2020/054542
- JP-A- 2008 058 519
- JP-A- 2008 058 519
- JP-A- 2010 057 726
- JP-A- 2012 100 899
- JP-A- 2012 100 899
- JP-A- 2017 080 389

## Description

### Technical Field

The present invention relates to an information processing device, an information processing method, and a computer program.

### Background Art

The radiologist uses mammography images and other medical images to determine the presence or absence of a lesion, etc. The presence or absence of the lesion is carefully determined by the radiologist. On the other hand, the radiologist may have to read a large number of medical images, and there is a small possibility that the lesion may be overlooked. For example, patent literature 1 discloses a technique for calculating risk, such as whether the lesion is physically present, by computer image analysis, for mammography images in which the potential lesion cannot be visually observed.

### Citation List

### Patent Literature

[Patent Literature 1] US 2017/0249739

### Summary of Invention

### Technical Problem

For example, the visibility of a medical image can vary depending on a factor such as the monitor used by the radiologist and the environment in which the radiologist reads the image. Then, the calculated risk should vary according to the factor. However, the technique described in patent literature 1 does not take the factor into account when calculating the above-mentioned risk, so the calculated risk deviates from the actual situation, and there is a high possibility that lesion in medical images will be overlooked.

The present invention has been made in view of the foregoing, and an object thereof is to provide an information processing device, an information processing method, and a computer program that can suppress overlooking of a lesion in a medical image.

### Solution to Problem

The present invention provides an information processing device as defined in claim 1.

According to the present invention, the data calculator generates the data that suppresses the overlooking of the lesion in the medical image data (the overlooking suppression data). Since the overlooking suppression data is based on the additional data including at least one of the monitor-related data and/or the environmental data, the overlooking suppression data takes into account the factor mentioned above, and as a result, the lesion in medical images can be suppressed from being overlooked.

Various embodiments of the present invention are described below. Any of the embodiments described below can be combined with one another.

Preferably, the overlooking suppression data includes image data indicating an area in the medical image data where the lesion is likely to be overlooked.

Preferably, the overlooking suppression data includes score data indicating possibility of the overlooking of the lesion in the medical image data.

Preferably, the overlooking suppression data includes location data, and the location data is data that specifies a location of an area in the medical image data where the lesion is likely to be overlooked.

Preferably, the monitor-related data includes at least one of a monitor set value, a monitor specification, a viewer set value, and/or a monitor measurement value, the monitor set value is a set value for defining the visibility of the image displayed on the display unit, the monitor specification indicates a characteristic of the monitor, the viewer set value is a set value for defining the visibility of the image displayed on the display unit and an application set value for displaying the image on the display unit, and the monitor measurement value is a luminance value or a chromaticity value of the display unit.

Preferably, the environmental data includes at least one of an illuminance value and /or a distance-measuring value, the illuminance value is a value indicating an illuminance around the display unit, and the distance-measuring value is a value indicating a distance between the monitor and a human body.

The present invention also provides an information processing method as defined in claim 7.

Furthermore, the present invention provides a computer program as defined in claim 8.

### Brief Description of Drawings

FIG. 1 is a functional block diagram of a first embodiment. FIG. 1 schematically illustrates the flow of various data in the operational phase of the information processing system 100.
FIG. 2 is a detailed functional block diagram of the data calculator 4 in the operational phase shown in FIG. 1.
FIG. 3 schematically illustrates the flow of various data in the learning phase of the information processing device 1.
FIG. 4 is a detailed functional block diagram of the data calculator 4 in the learning phase shown in FIG. 3.
FIG. 5 is a schematic diagram showing an example of the medical image data d2.
FIG. 6 is a schematic diagram showing an example of the probability map d21.
FIG. 7 is a schematic diagram showing an example of the candidate pixel map d22.
FIG. 8 is a schematic diagram showing an example of the overlooking area map d23.
FIG. 9 is a schematic diagram showing an example of the overlooking suppression data d10.
FIG. 10 is the modification 1 of the information processing device 1 according to the first embodiment. FIG. 10 schematically illustrates the flow of various data in the operational phase of the information processing system 100 according to the modification 1.
FIG. 11A is the overlooking area map d23 that schematically shows the line L for scoring. FIG. 11B shows a graph with the position of each pixel on line L as the horizontal axis and the probability P of each pixel on line L as the vertical axis.
FIG. 12 is a functional block diagram of the data calculator 4 and the monitor 21 according to the modification 4.
FIG. 13 is a functional block diagram of a second embodiment. FIG. 13 schematically illustrates the flow of various data in the operational phase of the monitor 21 (the information processing device).

### Description of Embodiments

Embodiments of the present invention will be described below. Any of features in the embodiments described below can be combined with one another.

### 1. FIRST EMBODIMENT

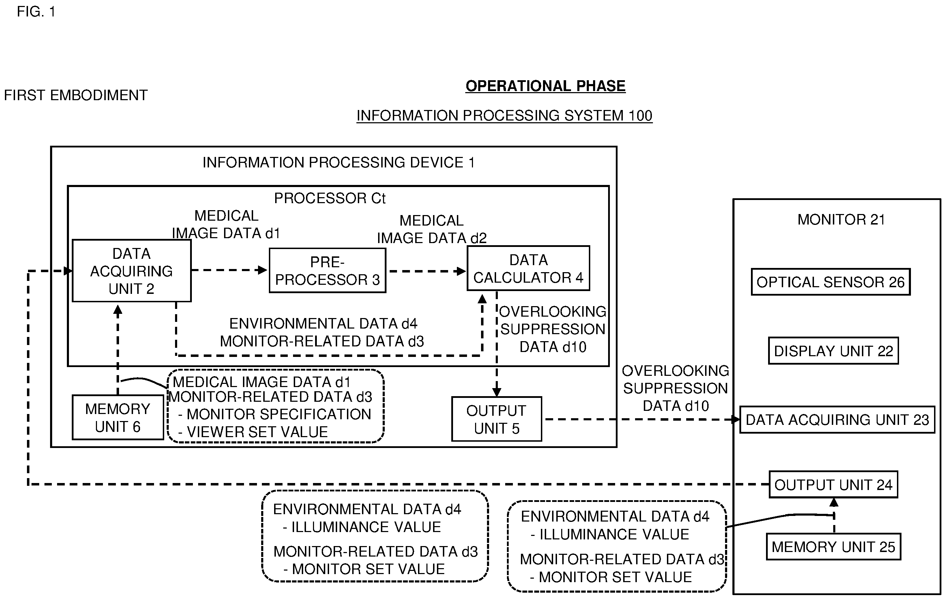

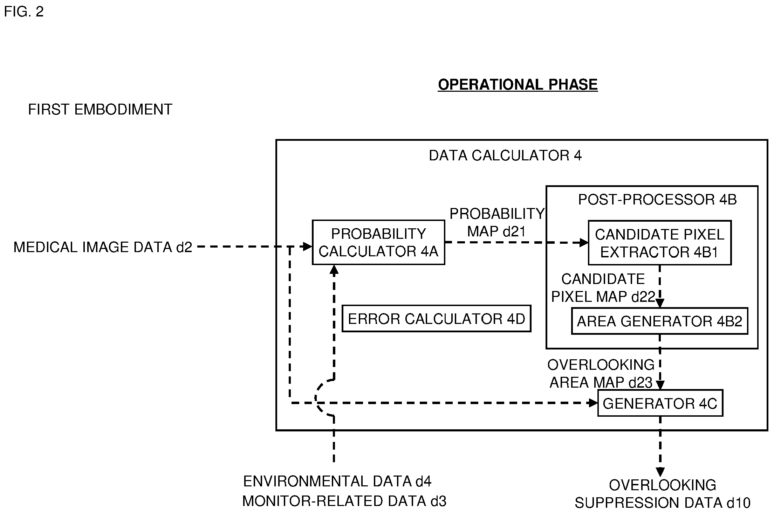

The information processing system 100 of the first embodiment includes an information processing device 1 and a monitor 21, as shown in FIG. 1. The information processing device 1 includes a processor Ct, an output unit 5, and a memory unit 6. The processor Ct includes a data acquiring unit 2, a pre-processor 3, and a data calculator 4. As shown in FIG.2, the data calculator 4 includes a probability calculator 4A, a post-processor 4B, and a generator 4C. The post-processor 4B includes a candidate pixel extractor 4B1 and an area generator 4B2.

Each of the above components may be realized by software or hardware. When realized by software, various functions can be realized by the CPU executing computer programs. The program may be stored in built-in memory or a non-transitory readable medium by a computer. Alternatively, the above functions are realized by reading the program stored in external memory using so-called cloud computing. When realized by hardware, the above functions can be performed by various circuits such as ASIC, FPGA, or DRP. The first embodiment deals with various information and concepts including this information, and the various information is a bit group of binary numbers having 0 or 1, and the various information is represented according to the level of signal value. And in the present embodiment, communications and calculations can be executed according to configurations of the above software and hardware.

The monitor 21 includes a display unit 22, a data acquiring unit 23, an output unit 24, a memory unit 25, and an optical sensor 26. The data acquiring unit 23 acquires the image data and other data processed by the information processing device 1, and the display unit 22 displays the image data acquired. The display unit 22 can be composed of, for example, an LCD monitor, CRT monitor, or OLED monitor.

### 1-1. SUMMARY OF DATA

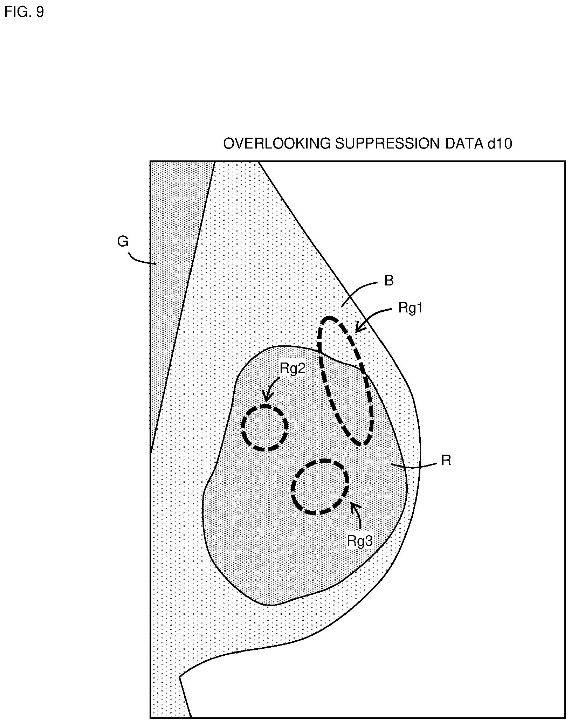

The processor Ct of the information processing device 1 is configured to acquire medical image data d1 and additional data and is configured to generate overlooking suppression data d10 in both the learning phase and the operational phase. The overlooking suppression data d10 includes the image data indicating the areas in the medical image data where the lesion is likely to be overlooked by the radiologist (corresponding to the overlooking areas Rg1 to Rg3 in FIG. 9). In other words, the overlooking suppression data d10 includes the image data indicating where the lesion is likely to be overlooked by the radiologist while reading. The radiologist's use of this overlooking suppression data d10 allows the radiologist to determine which area should be scrutinized particularly carefully, thus saving radiologist's concentration. And the information processing device 1 can suppress the decreasing of the attention of the radiologist during reading and, as a result, suppress the overlooking of the lesion in the medical images.

In the first embodiment, the overlooking suppression data d10 is image data that highlights the area where the lesion is likely to be overlooked, but it is not limited to this. The overlooking suppression data d10 may be, for example, image data in which the periphery of an area where the lesion is easily overlooked is surrounded by a highlighted line. That is, the area to be highlighted does not have to be the area itself where the lesion is easily overlooked, and may be wider than the area where the lesion is easily overlooked.

Here, various data used in the information processing device 1 and the monitor 21 will be described.

### 1-1-1. MEDICAL IMAGE DATA D1 AND D2

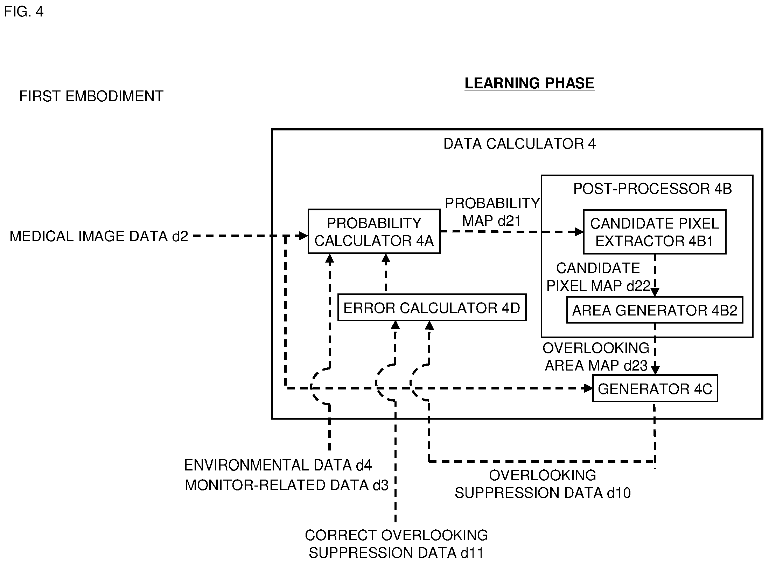

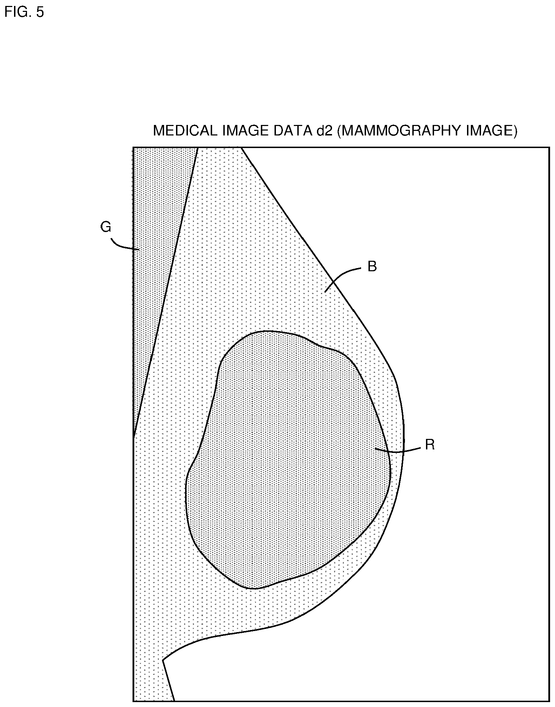

The medical image data d1 may be, for example, mammography image data, ultrasound image data, MRI image data, CT image data, chest X-ray image data, and angiographic image data. In the first embodiment, medical image data d1 is mammography image data (see FIG. 5). A mammography image is a digital image composed of many pixels. Each pixel has a pixel value. Mammography images usually include a pectoralis major muscle area G and a mammary area B, as shown in FIG. 4. The pectoralis major muscle area G is the area corresponding to the pectoralis major muscle, and the mammary area B is the area corresponding to the entire mammary area. The mammary area B includes a mammary gland area R. The mammary gland area R is a smaller area than the mammary area B. The mammary gland area R includes a mammary gland pixel and a fat pixel. The mammary gland pixel is a pixel corresponding to the mammary gland, and the fat pixel is a pixel corresponding to fat, other than a mammary gland pixel in the mammary gland area R. The mammary gland area R is the area that roughly encloses the mammary gland pixel.

In the pre-processor 3 of the processor Ct, the medical image data d1 is converted to the medical image data d2. The medical image data d2 is data to which the image size and the window level have been converted.

### 1-1-2. ADDITIONAL DATA

The additional data includes at least one of the monitor-related data d3 and/or the environmental data d4. In the first embodiment, the additional data includes both the monitor-related data d3 and the environmental data d4. The information processing device 1 performs processing using not only the medical image data d1 but also the additional data during the learning phase, so that the information processing device 1 performs machine learning in accordance with the radiologist's reading environment. In other words, the information processing device 1 is capable of machine learning more appropriately the area where the lesion is likely to be overlooked by the radiologist while taking into account the reading environment.

It is also possible that the information processing device can calculate the area where the lesion is likely to be overlooked based on the luminance of each pixel in the image data, without using machine learning. However, with such a method, only area of high luminance might be determined to be area where the lesion is likely to be overlooked. Not all areas, where the lesion is likely to be overlooked, are located in areas of high luminance. The information processing device 1 can learn to take into account not only the overlooking factor related to the luminance of the image data, but also the overlooking factor related to the radiologist's experience.

### 1-2-1-1. MONITOR-RELATED DATA d3

The monitor-related data d3 is data for defining the visibility of images displayed on the display unit 22 of the monitor 21. Then, the monitor-related data d3 includes at least one of a monitor set value, a monitor specification, and/or a viewer set value. In the first embodiment, the monitor-related data d3 includes three data which are the monitor set value, the monitor specification, and the viewer set value.

### <MONITOR SET VALUE>

The monitor set value is a set value for defining a visibility of the image displayed on the display unit 22.

The monitor set value may be, for example, a brightness set value, a gain set value, a contrast set value, a contrast ratio set value, a color temperature set value, a hue set value, a saturation set value, a sharpness set value, etc. The monitor set value includes at least one of these set values.

The brightness set value is a set value related to the brightness of the entire image. The brightness set value may include not only a brightness set value for the entire image, but also a brightness set value for a portion of the area (area of interest) as defined by the radiologist.

The gain set value is the luminance set value for red, green, and blue, respectively.

The contrast ratio set value is a set value that represents the difference between the luminance of the white area of the display and the luminance of the black area as a ratio. The contrast ratio set value may be a set value that represents the difference between the luminance of white displayed and the luminance of black displayed as a ratio.

The hue set value is a set value related to the hue of the image.

The sharpness set value is a set value related to the adjustment of the contour of the image.

### <MONITOR SPECIFICATION>

The monitor specification indicates the pre-existing characteristics of the monitor 21.

The monitor specification may be, for example, the glare characteristics and resolution of the monitor 21. The monitor specification includes at least one of glare characteristic and/or resolution.

The glare characteristic is a characteristic that indicates whether the display unit 22 of the monitor 21 is composed of a glare LCD or a non-glare LCD when the display unit 22 is an LCD monitor.

### <VIEWER SET VALUE>

The viewer set value is a set value for defining a visibility of an image displayed on the display unit 22. And the viewer set value is an application set value for displaying images on the display unit 22. This application is pre-stored in, for example, the information processing device 1.

The viewer set value may be, for example, a set value for black-and-white inversion processing, a set value for masking processing, a set value for gamma switching processing, a set value for equal magnification processing, a set value for pseudo-color processing, a set value for sharpening processing, and a set value for contrast enhancement processing. The viewer set value includes at least one of these set values.

The black-and-white inversion processing is an image processing that inverts black and white in an image.

The masking processing is an image processing that extracts only specific portions of the medical image data.

The gamma switching processing is image processing that switches the gamma value to correct the gamma characteristics.

The equal magnification processing is an image processing that equally magnifies pixels in a predefined area.

The pseudo-color processing is an image processing process that adds color to an image artificially.

The sharpening processing is an image processing that makes a blurred image clearer.

The contrast enhancement processing is an image processing that corrects brightness, gain, and gamma value, etc., depending on an image.

In the first embodiment, the viewer set value is described as a set value used in the application of information processing device 1, but it is not limited to this. It may be in the form that the monitor 21 has such an application and the monitor 21 determines the set value using said application.

### 1-1-2-2. ENVIRONMENTAL DATA D4

The environmental data d4 is data indicating an ambient environment of the monitor 21.

In the first embodiment, the environmental data d4 includes an illuminance value.

The illuminance value is a value indicating an illuminance around the display unit 22. In other words, the illuminance value corresponds to the illuminance in the space where the monitor 21 is located. In the first embodiment, the illuminance value can be acquired using the optical sensor 26 of the monitor 21.

### 1-2. CONFIGURATION OF INFORMATION PROCESSING DEVICE 1

The information processing device 1 has a processor Ct, an output unit 5, and a memory unit 6.

In the first embodiment, it will be described by assuming that the various types of data are processed by the information processing device 1 in both the operational phase and the learning phase. In the learning phase, an information processing device with higher computing power than the information processing device used in the operational phase may be used.

### 1-2-1. PROCESSOR Ct

The processor Ct has a data acquiring unit 2, a pre-processor 3, and a data calculator 4.

### 1-2-1-1. DATA ACQUIRING UNIT 2

As shown in FIG. 1, the data acquiring unit 2 is configured to acquire the medical image data d1 and the monitor-related data d3 (the monitor specification and the viewer set value) from the memory unit 6 in the operational phase. The data acquiring unit 2 is configured to acquire the monitor-related data d3 (the monitor set value) and the environmental data d4 (illuminance value) from the monitor 21 in the operational phase.

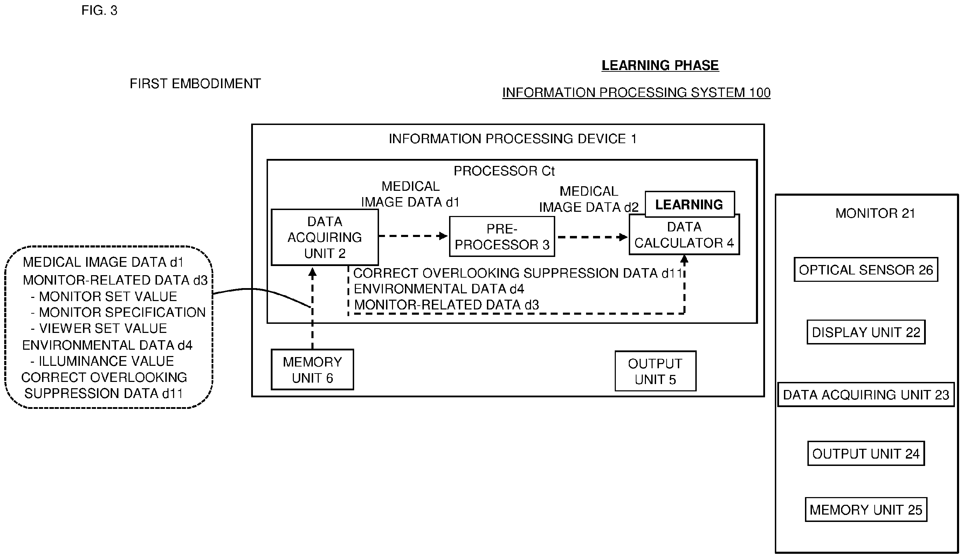

As shown in FIG. 3, the data acquiring unit 2 is also configured to acquire the medical image data d1, the monitor-related data d3 (the monitor specification, the viewer set value, and the monitor set value) and the environmental data d4 (the illuminance value) in the learning phase.

### 1-2-1-2. PRE-PROCESSOR 3

The pre-processor 3 performs various pre-processing operations for medical image data d1. The pre-processing is the processing performed to make the medical image data d1 suitable for processing by the data calculator 4. The pre-processor 3 converts from the medical image data d1 to the medical image data d2. The pre-processor 3 performs, for example, a size adjustment processing, a window level adjustment processing, and a noise removal processing. Some or all of these processing in the pre-processor 3 can be omitted if unnecessary.

In the size adjustment processing, the size of the medical image data d1 is adjusted. The medical image data d1 has different resolutions depending on the imaging equipment and settings. This means that the actual size per pixel varies depending on the input image. The size adjustment unit resizes each pixel to a predetermined size to remove fluctuations in detection accuracy due to difference in size per pixel.

The window level adjustment processing adjusts the window level of the medical image data d1. The window level adjustment is a processing to improve the contrast of a certain gradation range in an image with a wide range of gradation value. The window level adjustment can improve the visibility of the medical image data d1.

The noise removal processing performs noise removal of the medical image data d1. The medical image data d1 may include noise (for example, artificially-added labels) that reduces the accuracy with which the radiologist can analyze and extract the area in which lesion is likely to be overlooked. Therefore, the noise removal processing removes such noise.

### 1-2-1-3. DATA CALCULATOR 4

The data calculator 4 has a probability calculator 4A, a post-processor 4B, a generator 4C, and an error calculator 4D.

### <PROBABILITY CALCULATOR 4A>

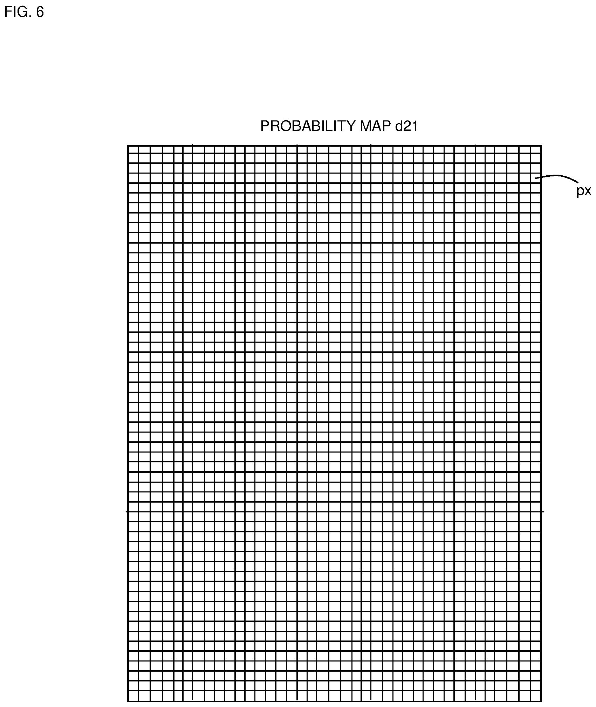

The probability calculator 4A calculates probability P for each pixel px in medical image data d2, respectively. Here, the probability P is a value indicating whether the area (pixel) corresponding to that probability P is the area (pixel) where the lesion is likely to be overlooked by the radiologist. Specifically, the probability calculator 4A generates a probability map d21 in which the probability P is specified for each pixel px, as shown in FIG. 6. In the first embodiment, the range of the probability map is over the entire area of the medical image data d2. The probability P is expressed, for example, as a value in the range 0 to 1. The higher the value of probability P, the more likely that the area (pixel) corresponding to the probability P is the area (pixel) where the lesion is easily overlooked by the radiologist.

The probability P is calculated based on a learning model that outputs the probability P when the medical image data d2 and the additional data are input. In the first embodiment, a fully convolutional network FCN (Fully Convolutional Network), a type of convolutional neural network, can be employed as the learning model (machine learning model) of the data calculator 4 (the probability calculator 4A). In the operational phase in FIG. 2, data calculator 4 has completed learning, while in the learning phase in FIG. 4, data calculator 4 is in the process of learning. In other words, in the operational phase, the filter weight coefficients of the neural network of the probability calculator 4A are already fixed, and in the learning phase, the filter weight coefficients of the neural network of the probability calculator 4A are not fixed and are updated as needed.

### <POST-PROCESSOR 4B>

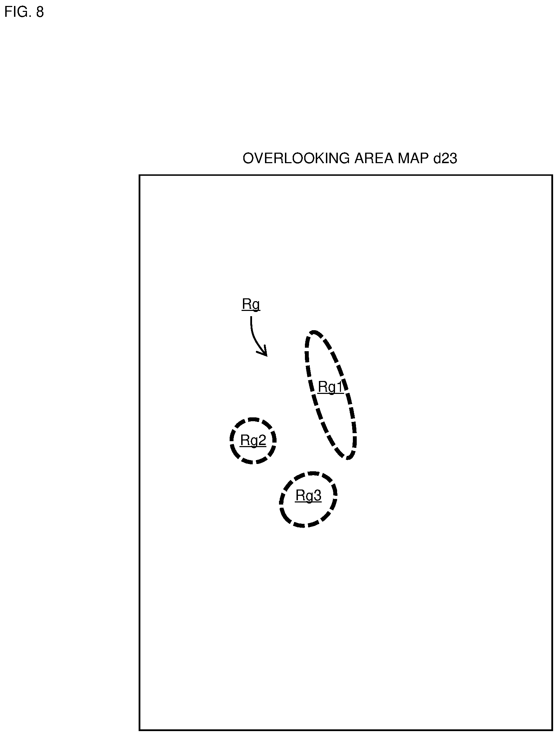

The post-processor 4B extracts an overlooking area Rg based on the probability P. The overlooking area Rg shows the area where the lesion is likely to be overlooked by the radiologist, as shown in FIG. 8. In the first embodiment, the overlooking area Rg includes three overlooking areas Rg1 to area Rg3. The post-processor 4B has a candidate pixel extractor 4B1 and an area generator 4B2.

### CANDIDATE PIXEL EXTRACTOR 4B1

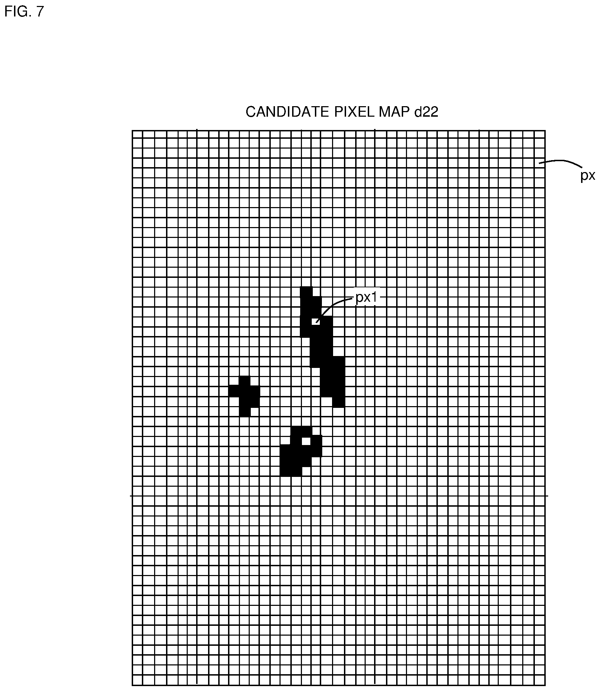

The candidate pixel extractor 4B1 performs threshold processing on the probability map d21. Specifically, the candidate pixel extractor 4B1 extracts as candidate pixels those whose pixel probability P in the probability map d2 1 is greater than the threshold value Th, generates the candidate pixel map d22 shown in FIG. 7, and outputs it to the area generator 4B2. In the first embodiment, the threshold value Th is a predetermined value. The threshold value Th may be a fixed value or a value that can be changed by the user as needed. The location of each pixel in candidate pixel map d22 corresponds to the location of each pixel in probability map d21. In the candidate pixel map d22, if the probability P of a pixel is equal to or greater than the threshold value Th, the value assigned to the pixel is 1, for example, and if the probability P of a pixel is less than the threshold value Th, the value assigned to the pixel is 0, for example. In FIG. 7, a pixel is indicated by a black dot if the value assigned to the pixel is 1, and a pixel is indicated by a white dot if the value assigned to the pixel is 0. The black dots are the candidate pixels.

### AREA GENERATOR 4B2

The area generator 4B2 performs missing area hole filling processing on the candidate pixel map d22 and forms the overlooking area Rg. Specifically, as shown in FIG. 7, a non-candidate pixel px1 may be present in the area where the candidate pixels are clustered. The presence of non-candidate pixel px1 complicates the shape of the overlooking area Rg and makes it difficult to specify the overlooking area. Therefore, the area generator 4B2 forms closed areas (the overlooking area Rg1 to the overlooking area Rg3) to fill the holes corresponding to the non-candidate pixel px1 (missing area). The missing area hole filling processing can be performed, for example, by filling holes between the start and end points of columns and rows, respectively. This allows area generator 4B2 to generate an overlooking area map d23 as shown in FIG. 8

### <GENERATOR 4C>

The generator 4C generates the overlooking suppression data d10 shown in FIG. 9 based on the medical image data d2 and the overlooking area map d23. Specifically, the generator 4C can generate the overlooking suppression data d10 by overlaying the overlooking area Rg of the overlooking area map d23 on the medical image data d2.

### <ERROR CALCULATOR 4D>

The error calculator 4D compares correct overlooking suppression data d11 with the overlooking suppression data d10 generated by the generator 4C, as shown in FIG. 4. In other words, the error calculator 4D calculates the error between the correct overlooking area and the calculated overlooking area. Here, the correct overlooking suppression data d11 is medical image data that indicates the area where the lesion is likely to be overlooked by the radiologist. In other words, the correct overlooking suppression data d11 is the medical image data that has highlighted area that is likely to be overlooked when the radiologist reads the corresponding medical images. The error calculator 4D outputs the calculated error to the probability calculator 4A. The probability calculator 4A updates the filter weight coefficients based on this error.

### 1-2-2. OUTPUT UNIT 5

The output unit 5 is configured to output the overlooking suppression data d10 generated by the generator 4C to the monitor 21.

### 1-2-3. MEMORY UNIT 6

Memory section 6 has a function to store various data. As shown in FIG. 1, the memory unit 6 stores the medical image data d1 and the monitor-related data d3 (the monitor specification and the viewer set value) in advance, which are used in the operation phase. As shown in FIG. 3, the medical image data d1, the monitor-related data d3 (the monitor set value, the monitor specification and the viewer set value) and the environmental data d4 (the illuminance value) used in the learning phase are stored in advance in the memory unit 6. Various data stored in the memory unit 6 are read out by the processor Ct.

### 1-3. CONFIGURATION OF MONITOR 21

The monitor 21 has a display unit 22, a data acquiring unit 23, an output unit 24, a memory unit 25, and an optical sensor 26.

### 1-3-1. DISPLAY UNIT 22

The display unit 22 has a function to display the data acquired by the data acquiring unit 23. Specifically, the display unit 22 can display overlooking suppression data d10. The radiologist displays and reads the overlooking suppression data d10 shown in FIG. 9 on the display unit 22. Here, the overlooking suppression data d10 is based on the monitor-related data d3 and the environmental data d4. Therefore, factors such as the monitor used by the radiologist and the environment in which the radiologist reads the image are taken into account in the overlooking suppression data d10, and as a result, the information processing device 1 according to the first embodiment can suppress the overlooking of the lesion in the medical image. By reading the overlooking suppression data d10 shown in Figure 9 on the display unit 22, the radiologist can determine which area of the medical image data d2 should be scrutinized particularly carefully, thus saving the radiologist's concentration.

### 1-3-2. DATA ACQUIRING UNIT 23

The data acquiring unit 23 is configured to acquire the overlooking suppression data d10, which is output from output unit 5.

### 1-3-3. OUTPUT UNIT 24

The output unit 24 is configured to output various data stored in the memory unit 25 to the information processing device 1.

### 1-3-4. MEMORY UNIT 25

The memory unit 25 has a function to store various data similarly to the memory unit 6. The memory unit 25 stores the monitor-related data d3 (the monitor set value), the environmental data d4 (the illuminance value) acquired by the optical sensor 26, etc.

### 1-3-5. OPTICAL SENSOR 26

The optical sensor 26 is configured to acquire the illuminance value (environmental data d4) of the light around the monitor 21 (the display unit 22).

### 1-4. DESCRIPTION of OPERATION

### 1-4-1. LEARNING PHASE

The operation of the information processing device 1 in the learning phase is described with reference to FIGS. 3 and 4.

The information processing method (the learning phase) of the first embodiment has an acquisition step and a calculation step.

The calculation step includes a pre-processing step, a probability map generation step (learning step), a candidate pixel generation step, an overlooking area map generation step, and an overlooking suppression data generation step.

In the acquisition step, the data acquiring unit 2 acquires the medical image data d1, the monitor related data d3 (monitor set value, monitor specification and viewer set value), the environmental data d4, and the correct overlooking suppression data d11.

In the pre-processing step, pre-processor 3 changes the size, etc. of medical image data d1 and generates the medical image data d2. In the probability map generation step, the probability calculator 4A generates the probability map d21 based on the medical image data d2, the monitor-related data d3 and the environmental data d4. This probability map generation step can also be called the learning step, since it is the step where machine learning is performed. In the probability map generation step (the learning step), the error calculated by error calculator 4D is input to probability calculator 4A. This error corresponds to the difference between the overlooking suppression data d10 acquired in the overlooking suppression data generation step described below and the correct overlooking suppression data d11. This allows the weight coefficients of the filter in the probability calculator 4A to be updated as needed, and increases the output accuracy of the probability calculator 4A. In other words, in the probability map generation step (learning step), the probability calculator 4A updates the filter weight coefficients as needed in the process of learning the relationship between inputs (the medical image data and the additional data) and outputs (the probability). In other words, the filter weight coefficients are updated to the value that better reflect the radiologist's experience. Then, the probability map d21 and the overlooking suppression data d10 get closer to the correct overlooking suppression data d11.

In the candidate pixel generation step, the candidate pixel extractor 4B1 performs threshold processing on the probability map d21 and generates the candidate pixel map d22. In the overlooking area map generation step, the area generator 4B2 performs missing area hole filling processing on the candidate pixel map d22 to form the overlooking area Rg, and generates the overlooking area map d23. In the overlooking suppression data generation step, the generator 4C generates the overlooking suppression data d10 based on the medical image data d2 and the overlooking area map d23.

### 1-4-2. OPERATIONAL PHASE

The operation in the operational phase is described based on FIGS. 1 and 2. The operation in the operational phase is described mainly on the part where it differs from the operation in the learning phase.

The information processing method (the operational phase) of the first embodiment has the acquisition step, the calculation step, and the output step.

The calculation step includes the pre-processing step, the probability map generation step, the candidate pixel generation step, the overlooking area map generation step, and the overlooking suppression data generation step.

In the acquisition step, the data acquiring unit 2 does not acquire the correct overlooking suppression data.

In the operational phase, the filter weight coefficients of the probability calculator 4A are fixed. In other words, the probability calculator 4A does not acquire the error from the error calculator 4D because the error is not calculated by the probability calculator 4A.

In the output step, the overlooking suppression data d10 is output to the display unit 22 of the monitor 21. This allows the radiologist to determine the location of the overlooking area Rg. In the first embodiment, the overlooking suppression data d10 is described as being image data, but it is not limited to this and may also be audio data. For example, the radiologist can determine the approximate location of the overlooking area Rg even if the location of the overlooking area Rg is output from the monitor 21's speaker in the output step.

### 1-5. MODIFICATION

### 1-5-1. MODIFICATION 1: FREQUENCY DATA GENERATOR 7

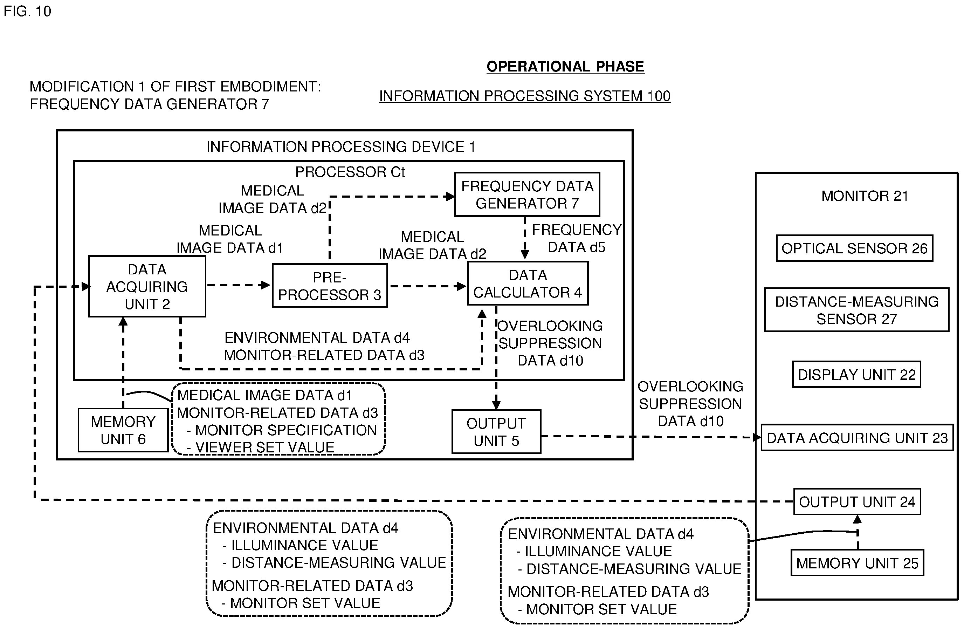

The information processing device 1 may be further provided with a frequency data generator 7, as shown in FIG. 10. The frequency data generator 7 performs the processing to acquire frequency data d5 that is data related to the frequency components of the medical image data d2. For example, the frequency data generator 7 can generate Fourier-transformed image data from the medical image data d2. The frequency data generator 7 can also perform a filtering process to extract specific frequencies in the medical image data d2 and generate image data from which edges are extracted. If the data calculator 4 learns the frequency data d5 in addition to the medical image data d2, etc., which is expected to have the effect that the information processing device 1 will generate more appropriate overlooking suppression data d10. This is because information on the frequency components of an image is considered relevant to the visibility of the image.

In the first embodiment, the environmental data d4 may have a distance-measuring value in addition to the illuminance value. Specifically, as shown in FIG. 10, the monitor 21 may have a distance-measuring sensor 27. The distance-measuring sensor 27 is configured to acquire a distance-measuring value. Here, the distance-measuring value is a value indicating the distance between the monitor 21 and the human body. The data calculator4 learns the distance-measuring value in addition to the frequency data d5, which is expected to have the effect that the information processing device 1 generates more appropriate overlooking suppression data d10. This is because the distance between the monitor 21 and the human body is considered relevant to the visibility of the image.

### 1-5-2. MODIFICATION 2: SCORE

In the first embodiment, the overlooking suppression data d10 is image data in which the overlooking area Rg, where the lesion is likely to be overlooked, is highlighted. In other words, in the first embodiment, the overlooking suppression data d10 is data that specifies the location of the overlooking area Rg where the lesion is likely to be overlooked. The manner of the overlooking suppression data d10 is not limited to specifying the location of the overlooking area Rg. The overlooking suppression data d10 may be a score (score data) indicating the possibility of overlooking of the lesion in the medical image data. The generator 4C calculates this score. The score may be displayed on the display unit 22 or output audibly. The higher this score, the more likely there is the area where the lesion is likely to be overlooked in the image. This score is based on the monitor-related data d3 and the environmental data d4, so it takes into account the factor such as the monitor used by the radiologist and the environment in which the radiologist reads. As a result, in the modification 2, as in the first embodiment, it is possible to suppress the overlooking of the lesion in the medical image. In addition, the radiologist can save the radiologist's concentration by referring to this score while reading.

The technique described in the patent literature 1 is the technique for calculating risk, such as whether the lesion is physically present. Therefore, when the calculated risk is the relatively small (the existence of the lesion has small possibility), the radiologist may be relaxed and the radiologist's attention decreases during the reading, resulting in the lesion in the medical image being overlooked. By contrast, in the modification 2, the radiologist needs to read the medical image data carefully even if the score is low, because the size of this score has no relationship to whether the lesion actually exists or not in the image. In other words, in the modification 2, even if this score is low, it avoids the decreasing of the attention of the radiologist.

### 1-5-2-1. SCORE CALCULATION METHOD 1

The score may be calculated by dividing the area of the overlooking area Rg by the area of the mammary gland area R. In general, the mammary gland area R is considered to be an area of high luminance and where lesion is more likely to be overlooked. Therefore, it is likely that much of the overlooking area Rg is included in the mammary gland area R. Thus, if the score is calculated based on the ratio of the area of the overlooking area Rg to the area of the mammary gland area R, then the score will reflect possibility of overlooking of the lesion in the medical image data.

The method for calculating the area of mammary gland area R is not limited. For example, the information processing device 1 can determine whether each pixel is the mammary gland pixel based on the luminance of each pixel, calculate the total number of mammary gland pixels, and use this total number as the area of the mammary gland area R.

The area of the overlooking area Rg can be the total number of pixels included in the overlooking area Rg.

### 1-5-2-2. SCORE CALCULATION METHOD 2

In the first embodiment, the overlooking area Rg includes three overlooking areas Rg1 to Rg3. The score may be calculated based on the area of the largest of area Rg1 to Rg3. The larger the area of the overlooking area, the more likely it is that the lesion will be overlooked. If the score is calculated based on the area of the largest area of the overlooking area Rg, then the score will reflect the possibility of overlooking of the lesion in the medical image data.

### 1-5-2-3. SCORE CALCULATION METHOD 3

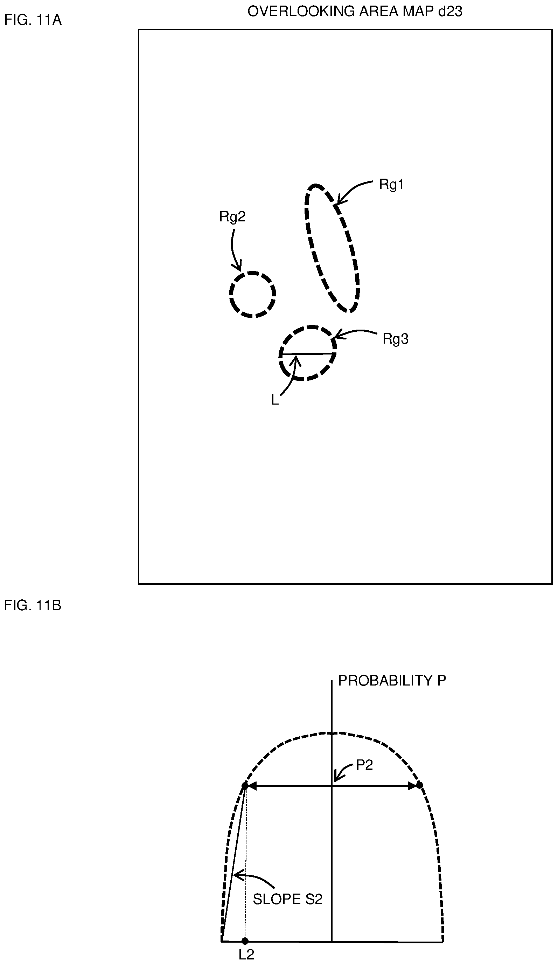

The score may be calculated based on the maximum width in a specific direction of the overlooking area Rg1 to area Rg3. Here, it is assumed that the specific direction is the left-right direction. As shown in FIG. 11A, the maximum width in the left-right direction is at the position of line L in the overlooking area Rg3. In other words, the score may be calculated based on the width (the length) of line L.

The score may be calculated based on the slope S2 of the line connecting the endpoints of line L in the graph shown in FIG. 11A and the coordinates of pixel L2 in the graph shown in FIG. 11B. Here, the pixel L2 is a pixel on the line L whose probability P is larger than the threshold value P2. The larger the slope S2, the more the overall shape of the graph tends to be convex upward. Therefore, the larger this slope S2 is, the higher the probability P of all pixels in the overlooking area Rg will be distributed.

If the score is calculated based on the width and slope S2 described above, then the score will reflect the possibility of overlooking of the lesion in the medical image data.

### 1-5-3. MODIFICATION 3: ACQUISITION OF MONITOR MEASUREMENT VALUE

In the first embodiment, it is explained that the monitor set value may be the brightness set value, but is not limited to this. The monitor-related data d3 may include the monitor measurement value. More specifically, the monitor-related data d3 may include at least one of the monitor set value, the monitor specification, the viewer set value, and/or the monitor measurement value.

The monitor measurement value is, for example, a luminance value or a chromaticity value. Since the monitor 21 includes an optical sensor (not shown) for measuring the luminance of the display unit 22, the information processing device 1 can use the luminance value acquired by this optical sensor instead of the brightness set value in the monitor set value.

### 1-5-4. MODIFICATION 4: VISUAL PROCESSING IN MONITOR 21

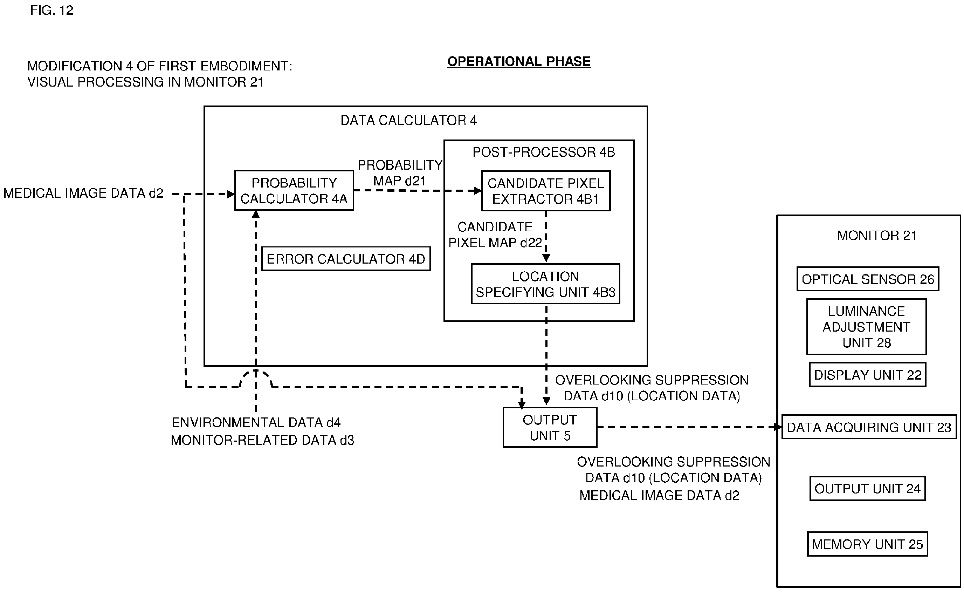

The first embodiment has the configuration in which the information processing device 1 performs visual processing to specify the overlooking areas Rg1 to Rg3, but it is not limited to this configuration. As shown in FIG. 12, the monitor 21 may perform the visual processing to specify the overlooking area. In the modification 4 shown in FIG. 12, the data calculator 4 does not have the generator 4C, and the data calculator 4 has a location specifying unit4B3 instead of the area generator 4B2. Further, the monitor 21 has a luminance adjustment unit 28.

The location specifying unit4B3 generates location data (the overlooking suppression data d10) that specifies the location of the overlooking area. For example, the location specifying unit4B3 can perform the missing area hole filling processing in the same manner as the area generator 4B2, and the location specifying unit4B3 generates location data (the overlooking suppression data d10) with the location data of the candidate pixels specified in the candidate pixel map d22 and the location data of the pixels filled by the missing area hole filling processing. That is, this generated location data (the overlooking suppression data d10) is the location data that specifies the location of the area of the medical image data d2 where the lesion is likely to be overlooked. The location specifying unit 4B3 outputs this generated location data to the output unit 5.

Based on this location data and the medical image data d2, the luminance adjustment unit 28 highlights the pixel (the area) in the medical image data d2 where the lesion is likely to be overlooked. Specifically, the luminance adjustment unit 28 has the function of increasing the luminance value of the pixel corresponding to the position data when the monitor 21 displays the medical image data d2. In other words, the luminance value of the pixel corresponding to the position data is adjusted from the luminance value in the medical image data d2 to the luminance value larger than the said value. The luminance adjustment unit 28 may highlight pixels where the lesion is likely to be overlooked by reducing relatively the luminance value of pixels surrounding the pixel corresponding to this location data.

### 2. SECOND EMBODIMENT

In the second embodiment, descriptions of configurations common to the first embodiment will be omitted as needed, and the description will focus on configurations that differ.

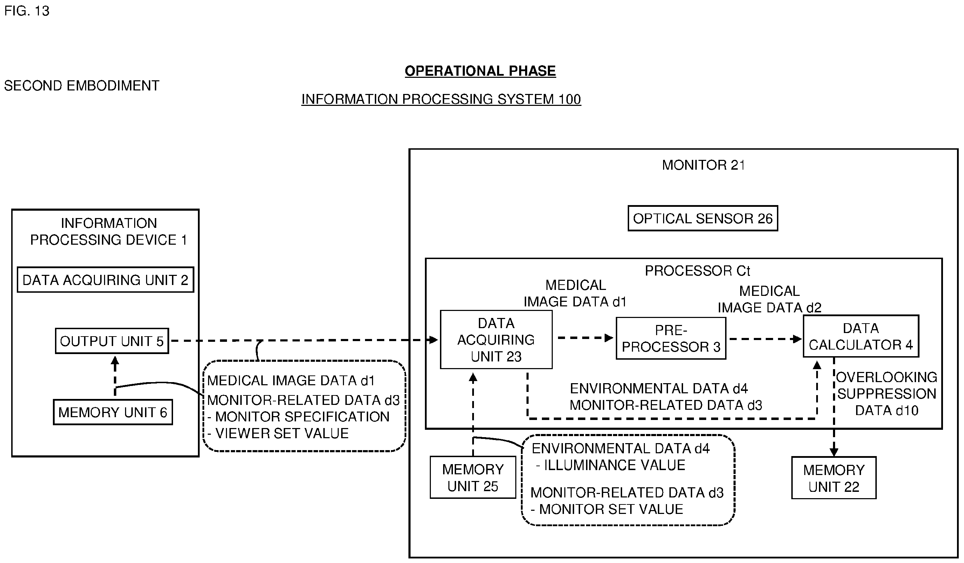

The first embodiment has the configuration in which the information processing device 1 has the data calculator 4, but it is not limited to this configuration. In the second embodiment, the monitor 21 includes the processor Ct (the data calculator 4), as shown in FIG. 13. In other words, in the second embodiment, the monitor 21 functions as an information processing device that calculates the overlooking suppression data d10. The second embodiment also has the same effects as those in the first embodiment.

### Description of Reference Signs

1: information processing device
2: data acquiring unit
3: pre-processor
4: data calculator
4A: probability calculator
4B: post-processor
4B1: candidate pixel extractor
4B2: area generator
4B3: location specifying unit
4C: generator
4D: error calculator
5: output unit
6: memory unit
7: frequency data generator
21: monitor
22: display unit
23: data acquiring unit
24: output unit
25: memory unit
26: optical sensor
27: distance-measuring sensor
100: information processing system
Ct: processor
B: mammary area
G: pectoralis major muscle area
R: mammary gland area
Rg: overlooking area
Rg1: overlooking area
Rg 2: overlooking area
Rg 3: overlooking area
d1: medical image data (data before pre-processing)
d2: medical image data (data after pre-processing)
d3: monitor-related data
d4: environmental data
d10: overlooking suppression data
d11: correct overlooking suppression data
d21: probability map
d22: candidate pixel map
d23: area map

## Claims

1. An information processing device comprising:
a data acquiring unit; and a data calculator, wherein
the data acquiring unit is configured to acquire medical image data and additional data,
the additional data includes at least one of monitor-related data and/or environmental data,
the monitor-related data is data for defining a visibility of an image displayed on a display unit of a monitor,
the environmental data is data indicating an ambient environment of the monitor, and
the data calculator is configured to calculate a probability based on a learning model that outputs the probability when the medical image data and the additional data are input, and is configured to generate overlooking suppression data based on the probability,
the overlooking suppression data is data that suppresses overlooking of a lesion in the medical image data, and
the probability is a value indicating whether the lesion is likely to be overlooked.

2. The information processing device of Claim 1 wherein,
the overlooking suppression data includes image data indicating an area in the medical image data where the lesion is likely to be overlooked.

3. The information processing device of Claim 1 or 2 wherein,
the overlooking suppression data includes score data indicating possibility of the overlooking of the lesion in the medical image data.

4. The information processing device of any one of Claims 1 to 3 wherein,
the overlooking suppression data includes location data, and
the location data is data that specifies a location of an area in the medical image data where the lesion is likely to be overlooked.

5. The information processing device of any one of Claims 1 to 4 wherein,
the monitor-related data includes at least one of a monitor set value, a monitor specification, a viewer set value, and/or a monitor measurement value,
the monitor set value is a set value for defining the visibility of the image displayed on the display unit,
the monitor specification indicates a characteristic of the monitor,
the viewer set value is a set value for defining the visibility of the image displayed on the display unit and an application set value for displaying the image on the display unit, and
the monitor measurement value is a luminance value or a chromaticity value of the display unit.

6. The information processing device of any one of Claims 1 to 5 wherein,
the environmental data includes at least one of an illuminance value and/or a distance-measuring value,
the illuminance value is a value indicating an illuminance around the display unit, and
the distance-measuring value is a value indicating a distance between the monitor and a human body.

7. An information processing method comprising:
acquisition step; and calculation step, wherein
in the acquisition step, medical image data and additional data are acquired,
the additional data includes at least one of monitor-related data and/or environmental data,
the monitor-related data is data for defining a visibility of an image displayed on a display unit of a monitor,
the environmental data is data indicating an ambient environment of the monitor, and
in the calculation step, a probability is calculated based on a learning model that outputs the probability when the medical image data and the additional data are input, and overlooking suppression data is generated based on the probability,
the overlooking suppression data is data that suppresses overlooking of a lesion in the medical image data, and
the probability is a value indicating whether the lesion is likely to be overlooked.

8. A computer program causing a computer to execute an information processing method, the information processing method comprising:
acquisition step; and calculation step, wherein
in the acquisition step, medical image data and additional data are acquired,
the additional data includes at least one of monitor-related data and/or environmental data,
the monitor-related data is data for defining a visibility of an image displayed on a display unit of a monitor,
the environmental data is data indicating an ambient environment of the monitor, and
in the calculation step, a probability is calculated based on a learning model that outputs the probability when the medical image data and the additional data are input, and overlooking suppression data is generated based on the probability,
the overlooking suppression data is data that suppresses overlooking of a lesion in the medical image data, and
the probability is a value indicating whether the lesion is likely to be overlooked.

## Patentansprüche

1. Informationsverarbeitungsvorrichtung umfassend:
eine Datenerfassungseinheit und einen Datenrechner, wobei
die Datenerfassungseinheit konfiguriert ist, medizinische Bilddaten und zusätzliche Daten zu erfassen,
die zusätzlichen Daten mindestens eines von überwachungsbezogenen Daten und/oder Umgebungsdaten einschließen,
die überwachungsbezogenen Daten Daten zum Definieren einer Sichtbarkeit eines auf einer Displayeinheit eines Monitors angezeigten Bildes sind,
die Umgebungsdaten Daten sind, die nähere Umgebungsverhältnisse des Monitors anzeigen, und
der Datenrechner konfiguriert ist, eine Wahrscheinlichkeit auf der Basis eines Lernmodells zu berechnen, das die Wahrscheinlichkeit ausgibt, wenn die medizinischen Bilddaten und die zusätzlichen Daten eingegeben werden, und konfiguriert ist, auf der Basis der Wahrscheinlichkeit Unterdrückungsdaten für versehentliches Übersehen zu erzeugen,
wobei die Unterdrückungsdaten für versehentliches Übersehen Daten sind, die versehentliches Übersehen einer Wunde in den medizinischen Bilddaten unterdrücken, und
die Wahrscheinlichkeit ein Wert ist, der anzeigt, ob die Wunde wahrscheinlich versehentlich übersehen wird.

2. Informationsverarbeitungsvorrichtung nach Anspruch 1, wobei
die Unterdrückungsdaten für versehentliches Übersehen Bilddaten einschließen, die einen Bereich in den medizinischen Bilddaten anzeigen, wo die Wunde wahrscheinlich versehentlich übersehen wird.

3. Informationsverarbeitungsvorrichtung nach Anspruch 1 oder 2, wobei
die Unterdrückungsdaten für versehentliches Übersehen Bewertungsdaten einschließen, die die Möglichkeit des versehentlichen Übersehens der Wunde in den medizinischen Bilddaten anzeigen.

4. Informationsverarbeitungsvorrichtung nach einem der Ansprüche 1 bis 3, wobei
die Unterdrückungsdaten für versehentliches Übersehen Ortungsdaten einschließen, und
die Ortungsdaten Daten sind, die einen Ort für einen Bereich in den medizinischen Bilddaten spezifizieren, wo die Wunde wahrscheinlich versehentlich übersehen wird.

5. Informationsverarbeitungsvorrichtung nach einem der Ansprüche 1 bis 4, wobei
die überwachungsbezogenen Daten mindestens eines der Folgenden einschließen: einen Monitoreinstellwert, eine Monitorspezifikation, einen Betrachtereinstellwert und/oder einen Monitormesswert,
der Monitoreinstellwert ein Einstellwert zum Definieren der Sichtbarkeit des auf der Displayeinheit angezeigten Bildes ist,
die Monitorspezifikation eine Eigenschaft des Monitors anzeigt,
der Betrachtereinstellwert ein Einstellwert zum Definieren der Sichtbarkeit des auf der Displayeinheit angezeigten Bildes und ein Anwendungseinstellwert zum Anzeigen des Bildes auf der Displayeinheit ist, und
der Monitormesswert ein Luminanzwert oder ein Chromatizitätswert der Displayeinheit ist.

6. Informationsverarbeitungsvorrichtung nach einem der Ansprüche 1 bis 5, wobei
die Umgebungsdaten mindestens einen von einem Illuminanzwert und/oder einem Abstandsmesswert einschließen,
wobei der Illuminanzwert ein Wert ist, der ein Beleuchtungsmaß um die Displayeinheit herum anzeigt, und
der Abstandsmesswert ein Wert ist, der einen Abstand zwischen dem Monitor und einem menschlichen Körper anzeigt.

7. Informationsverarbeitungsverfahren umfassend:
einen Datenerfassungsschritt und einen Berechnungsschritt, wobei
im Datenerfassungsschritt medizinische Bilddaten und zusätzliche Daten erfasst werden,
die zusätzlichen Daten mindestens eines von überwachungsbezogenen Daten und/oder Umgebungsdaten einschließen,
die überwachungsbezogenen Daten Daten zum Definieren einer Sichtbarkeit eines auf einer Displayeinheit eines Monitors angezeigten Bildes sind,
die Umgebungsdaten Daten sind, die nähere Umgebungsverhältnisse des Monitors anzeigen, und
im Berechnungsschritt eine Wahrscheinlichkeit auf der Basis eines Lernmodells berechnet wird, das die Wahrscheinlichkeit ausgibt, wenn die medizinischen Bilddaten und die zusätzlichen Daten eingegeben werden, und auf der Basis der Wahrscheinlichkeit Unterdrückungsdaten für versehentliches Übersehen erzeugt werden,
wobei die Unterdrückungsdaten für versehentliches Übersehen Daten sind, die versehentliches Übersehen einer Wunde in den medizinischen Bilddaten unterdrücken, und
die Wahrscheinlichkeit ein Wert ist, der anzeigt, ob die Wunde wahrscheinlich versehentlich übersehen wird.

8. Computerprogramm, das einen Computer veranlasst, ein Informationsverarbeitungsverfahren auszuführen, wobei das Informationsverarbeitungsverfahren Folgendes umfasst:
einen Datenerfassungsschritt und einen Berechnungsschritt, wobei
im Datenerfassungsschritt medizinische Bilddaten und zusätzliche Daten erfasst werden,
die zusätzlichen Daten mindestens eines von überwachungsbezogenen Daten und/oder Umgebungsdaten einschließen,
die überwachungsbezogenen Daten Daten zum Definieren einer Sichtbarkeit eines auf einer Displayeinheit eines Monitors angezeigten Bildes sind,
die Umgebungsdaten Daten sind, die nähere Umgebungsverhältnisse des Monitors anzeigen, und
im Berechnungsschritt eine Wahrscheinlichkeit auf der Basis eines Lernmodells berechnet wird, das die Wahrscheinlichkeit ausgibt, wenn die medizinischen Bilddaten und die zusätzlichen Daten eingegeben werden, und auf der Basis der Wahrscheinlichkeit Unterdrückungsdaten für versehentliches Übersehen erzeugt werden,
wobei die Unterdrückungsdaten für versehentliches Übersehen Daten sind, die versehentliches Übersehen einer Wunde in den medizinischen Bilddaten unterdrücken, und
die Wahrscheinlichkeit ein Wert ist, der anzeigt, ob die Wunde wahrscheinlich versehentlich übersehen wird.

## Revendications

1. Dispositif de traitement d'informations comprenant :
une unité d'acquisition de données ; et un calculateur de données, dans lequel
l'unité d'acquisition de données est configurée pour acquérir des données d'image médicale et des données supplémentaires,
les données supplémentaires comprennent au moins un élément parmi des données relatives au moniteur et/ou des données relatives à l'environnement,
les données relatives au moniteur sont des données définissant une visibilité d'une image affichée sur une unité d'affichage d'un moniteur,
les données relatives à l'environnement sont des données indiquant un environnement ambiant du moniteur, et
le calculateur de données est configuré pour calculer une probabilité sur la base d'un modèle d'apprentissage qui délivre la probabilité lorsque les données d'image médicale et les données supplémentaires sont entrées, et est configuré pour générer des données de suppression de négligence sur la base de la probabilité,
les données de suppression de négligence sont des données qui suppriment la négligence d'une lésion dans les données d'image médicale, et
la probabilité est une valeur indiquant si la lésion est susceptible d'être négligée.

2. Dispositif de traitement d'informations selon la revendication 1 dans lequel
les données de suppression de négligence comprennent des données d'image indiquant une zone dans les données d'image médicale dans laquelle la lésion est susceptible d'être négligée.

3. Dispositif de traitement d'informations selon la revendication 1 ou 2, dans lequel
les données de suppression de négligence comprennent des données de score indiquant une possibilité de négliger la lésion dans les données d'image médicale.

4. Dispositif de traitement d'informations selon l'une quelconque des revendications 1 à 3, dans lequel
les données de suppression de négligence comprennent des données d'emplacement, et
les données d'emplacement sont des données qui spécifient un emplacement d'une zone dans les données d'image médicale dans laquelle la lésion est susceptible d'être négligée.

5. Dispositif de traitement d'informations selon l'une quelconque des revendications 1 à 4, dans lequel
les données relatives au moniteur comprennent au moins un élément parmi une valeur prédéterminée de moniteur, une spécification de moniteur, une valeur prédéterminée de visualiseur, et/ou une valeur de mesure de moniteur,
la valeur prédéterminée de moniteur est une valeur prédéterminée pour définir la visibilité de l'image affichée sur l'unité d'affichage,
la spécification de moniteur indique une caractéristique du moniteur,
la valeur prédéterminée de visualiseur est une valeur prédéterminée pour définir la visibilité de l'image affichée sur l'unité d'affichage et une valeur prédéterminée d'application pour afficher l'image sur l'unité d'affichage, et
la valeur de mesure de moniteur est une valeur de luminance ou une valeur de chromaticité de l'unité d'affichage.

6. Dispositif de traitement d'informations selon l'une quelconque des revendications 1 à 5, dans lequel
les données relatives à l'environnement comprennent au moins un élément parmi une valeur d'éclairement et/ou une valeur de mesure de distance,
la valeur d'éclairement est une valeur indiquant un éclairement autour de l'unité d'affichage, et
la valeur de mesure de distance est une valeur indiquant une distance entre le moniteur et un corps humain.

7. Procédé de traitement d'informations comprenant :
une étape d'acquisition ; et une étape de calcul, dans lequel
dans l'étape d'acquisition, des données d'image médicale et des données supplémentaires sont acquises,
les données supplémentaires comprennent au moins un élément parmi des données relatives au moniteur et/ou des données relatives à l'environnement,
les données relatives au moniteur sont des données définissant une visibilité d'une image affichée sur une unité d'affichage d'un moniteur,
les données relatives à l'environnement sont des données indiquant un environnement ambiant du moniteur, et
dans l'étape de calcul, une probabilité est calculée sur la base d'un modèle d'apprentissage qui délivre la probabilité lorsque les données d'image médicale et les données supplémentaires sont entrées, et des données de suppression de négligence sont générées sur la base de la probabilité,
les données de suppression de négligence sont des données qui suppriment la négligence d'une lésion dans les données d'image médicale, et
la probabilité est une valeur indiquant si la lésion est susceptible d'être négligée.

8. Programme informatique amenant un ordinateur à exécuter un procédé de traitement d'informations, le procédé de traitement d'informations comprenant :
une étape d'acquisition ; et une étape de calcul, dans lequel
dans l'étape d'acquisition, des données d'image médicale et des données supplémentaires sont acquises,
les données supplémentaires comprennent au moins un élément parmi des données relatives au moniteur et/ou des données relatives à l'environnement,
les données relatives au moniteur sont des données définissant une visibilité d'une image affichée sur une unité d'affichage d'un moniteur,
les données relatives à l'environnement sont des données indiquant un environnement ambiant du moniteur, et
dans l'étape de calcul, une probabilité est calculée sur la base d'un modèle d'apprentissage qui délivre la probabilité lorsque les données d'image médicale et les données supplémentaires sont entrées, et des données de suppression de négligence sont générées sur la base de la probabilité,
les données de suppression de négligence sont des données qui suppriment la négligence d'une lésion dans les données d'image médicale, et
la probabilité est une valeur indiquant si la lésion est susceptible d'être négligée.
